# EUROPEAN PATENT APPLICATION

(11) **EP 4 296 287 A1**
(43) Date of publication of application: **27.12.2023**
(21) Application number: 22875043.6
(22) Date of filing: 29.09.2022
(51) Int. Cl.: C07K 19/00, A61P 37/00

(54) **METHOD FOR TREATING SJOGREN'S SYNDROME USING TACI-FC FUSION PROTEIN**

(30) Priority: 30.09.2021 CN 202111156909
(71) Applicant: RemeGen Co., Ltd., Yantai, Shandong 264006 (CN)
(72) Inventor: FANG, Jianmin, Shandong 264006 (CN); WANG, Wenxiang, Shandong 264006 (CN)
(74) Representative: HGF
(86) International application number: PCT/CN2022/122399
(87) International publication number: WO 2023/051660

(57) **Abstract**

Provided are a drug for treating Sjogren's syndrome using a TACI-Fc fusion protein, and a dosage regimen, a dosing interval and an administration mode thereof. The results show that the provided TACI-Fc fusion protein significantly improves the ESDAI score and the MF-20 score at 24 weeks in a patient with Sjogren's syndrome, and same exhibits good security during treatment.

## Description

### FIELD

The present disclosure relates to a drug for treating Sjogren's syndrome, and a dosage regimen, a dosing interval and an administration mode thereof.

### BACKGROUND

Sjogren's syndrome (SS) is a chronic autoimmune disease mainly invading exocrine glands such as lacrimal and salivary glands, thus also known as autoimmune exocrine gland disease. It has manifestations mainly including keratoconjunctivitis sicca, conjunctivitis, and xerostomia, and can affect other systems such as respiratory system, digestive system, urinary system, blood system, nervous system, muscles, and joints, causing damage to multiple systems and organs.

Sjogren's syndrome is a global disease with a high incidence. Most of the patients are middle-aged and elderly people aged 40 to 60, of whom more than 90% are women and few are children. Due to the lack of uniform diagnostic criteria so far, the prevalence of the disease is very inaccurate, generally estimated at 0.1%-0.7%. In the United States, Sjogren's syndrome is second only to rheumatoid arthritis in prevalence. According to a survey of more than 10,000 people in China, the prevalence of this disease is 0.29%-0.77%, which indicates that the prevalence of this disease in China is not lower than the incidence of rheumatoid arthritis of 0.3%-0.4%.

At present, the treatment of Sjogren's syndrome is mainly to suppress the abnormal immune response in patients through hormones, thereby protecting the functions of exocrine glands and other important organs. However, there is no specific therapy or biological drug approved for Sjogren's syndrome in the world. As of July 20, 2021, there are only five new biological drugs targeting BAFF/BLyS/APRIL in the clinical research for Sjogren's syndrome (see Table 1).

**Table 1 New biological drugs targeting BAFF/BLyS/APRIL in clinical research for Sjogren's syndrome worldwide**

| **Drug name** | **Research institute** | **Highest research and development status in the world for related indications** |
|---|---|---|
| Telitacicept | RemeGen Biopharmaceutical (Yantai) Co., Ltd. | Clinical phase II |
| Ianalumab | Novartis | Clinical phase II |
| ALPN-303 | Alpine Immune Sciences; | Preclinical studies |
| Briobacept | Biogen (Denmark) A/S; Genentech | Clinical phase II |
| Tibulizumab (Eli Lilly) | Eli Lilly | Clinical phase I |

Therefore, there is a huge unmet clinical need in the field of Sjogren's syndrome treatment, both in China and globally.

### SUMMARY

Through in-depth analysis of a large number of clinical data, the present disclosure surprisingly found that the TACI-Fc fusion protein provided by the present disclosure produced unexpected technical effects in treating cancer patients with Sjogren's syndrome. Specifically, the present disclosure provides a method for treating Sjogren's syndrome, comprising administering a therapeutically effective amount of TACI-Fc fusion protein to a patient with Sjogren's syndrome, wherein the TACI-Fc fusion protein comprises:
(i) a TACI extracellular region or a fragment thereof binding to Blys and/or APRII,; and
(ii) a fragment of human immunoglobulin constant region.

In some preferred embodiments, the TACI-Fc fusion protein is administered at a single dose of about 0.1 to 10 mg/kg.

The present disclosure further provides use of a TACI-Fc fusion protein in the manufacture of a medicament for treating Sjogren's syndrome.

In one embodiment, the TACI extracellular region or the fragment thereof comprises an amino acid sequence set forth in SEQ ID NO: 1.
**SEQ ID NO:1**

In one embodiment, the human immunoglobulin is IgG1, or the fragment of human immunoglobulin constant region comprises an amino acid sequence set forth in SEQ ID NO: 2 or an amino acid sequence having at least 90%, at least 91%, at least 92%, at least 93%, at least 95%, or at least 96% identity to SEQ ID NO: 2.
**SEQ ID NO:2**

In a preferred embodiment, the fragment of human immunoglobulin constant region comprises one or more modifications of amino acid at positions 3, 8, 14, 15, 17, 110, 111 or 173 of SEQ ID NO: 2, wherein the modification is preferably substitution, deletion or insertion of amino acid.

In some specific embodiments, the substitution is selected from the group consisting of P3T, L8P, L14A, L15E, G17A, A110S, P111S and A173T.

In a specific embodiment, the fragment of human immunoglobulin constant region comprises an amino acid sequence set forth in SEQ ID NO: 3.
**SEQ ID NO:3**

In one embodiment, the TACI-Fc fusion protein is Telitacicept, and has an amino acid sequence set forth in SEQ ID NO: 4.
**SEQ ID NO:4**

In some preferred embodiments, the TACI-Fc fusion protein is administered subcutaneously, intramuscularly or intravenously, or at the thigh, abdomen or upper arm.

In some preferred embodiments, the TACI-Fc fusion protein is administered 2-4 times at intervals of one month.

In some preferred embodiments, the TACI-Fc fusion protein is administered at a frequency of once a week.

In some preferred embodiments, the administration lasts for about 2-50 weeks.

In some preferred embodiments, the Sjogren's syndrome is primary Sjogren's syndrome.

In some preferred embodiments, the Sjogren's syndrome is secondary Sjogren's syndrome.

In some preferred embodiments, the TACI-Fc fusion protein is administered at a single dose of 160 to 240 mg, preferably 160 mg or 240 mg.

The present disclosure further includes use of the above TACI-Fc fusion protein in the manufacture of a medicament for treating Sjogren's syndrome.

The results of a clinical study carried out by the present disclosure show that Telitacicept treatment significantly improved the ESSDAI score and MF-20 score of pSS patients at the 24th week, and Telitacicept showed good safety in pSS patients.

### BRIEF DESCRIPTION OF DRAWINGS

FIG. 1 shows the change rate of IgG levels in the placebo group, 160 mg group, and 240 mg group compared with the baseline;
FIG. 2 shows the change rate of IgA levels in the placebo group, 160 mg group, and 240 mg group compared with the baseline;
FIG. 3 shows the change rate of IgM levels in the placebo group, 160 mg group, and 240 mg group compared with the baseline;
FIG. 4 shows the change rate of CD19+ B cell counts in the placebo group, 160 mg group, and 240 mg group compared with the baseline;
FIG. 5 shows the change rate of CD4+ T cell counts in the placebo group, 160 mg group, and 240 mg group compared with the baseline; and
FIG. 6 shows the change rate of CD8+ T cell counts in the placebo group, 160 mg group, and 240 mg group compared with the baseline.

### DETAILED DESCRIPTION

Unless otherwise defined, all scientific and technical terms used herein have the same meaning as understood by those of ordinary skill in the art. For definitions and terms in the art, those skilled can make a reference to Current Protocols in Molecular Biology (Ausubel).

The three-letter codes and one-letter codes of amino acids used in the present disclosure are as described in J.biol.chem, 243, p3558 (1968).

The present disclosure provides use of transmembrane activator, calcium regulator and cyclophilin ligand interactor (TACI)-immunoglobulin fusion protein (i.e., TACI-Fc fusion protein) in the treatment of Sjogren's syndrome. The patients in the present disclosure are preferably mammals, such as humans.

The term "TACI" in the present disclosure refers to transmembrane activator and CAML interactor, which is a member of the tumor necrosis factor receptor superfamily. The term "BLys" in the present disclosure refers to B lymphocyte stimulator, which is a member of the TNF ligand superfamily existing in two forms of membrane-bound and soluble forms, expressed on the surface of bone marrow cells, and selectively stimulates the proliferation of B lymphocytes and the production of immunoglobulin. The term "APRIL" (a proliferation-inducing ligand) in the present disclosure refers to a tumor necrosis factor (TNF) analogue, which can stimulate the proliferation of primitive B cells and T cells in the body, promote the accumulation of B cells and increase their content in spleen. APRIL can specifically bind to TACI and BCMA, and the binding can prevent APRIL from binding to B cells and thus inhibit the proliferative response of primitive B cells stimulated by APRIL. Moreover, APRIL can compete with BLys for binding to receptors (BCMA and TACI).

The term "TACI-Fc fusion protein" involved in the present disclosure refers to transmembrane activator, calcium regulator and cyclophilin ligand interactor (TACI)-immunoglobulin fusion protein (i.e., TACI-Fc fusion protein). The TACI-immunoglobulin fusion protein provided by the present disclosure comprises: (i) a TACI extracellular region or a fragment thereof binding to Blys and/or APRIL; and (ii) a fragment of human immunoglobulin constant region.

For the term "TACI extracellular region or a fragment thereof binding to Blys and/or APRIL", reference can be made to the TACI extracellular domain and the specific fragment of TACI extracellular domain capable of interacting with TACI ligands disclosed in US Patent Nos. 5,969,102, 6,316,222 and 6,500,428 and US Patent Application Nos. 09/569,245 and 09/627,206, the contents of which are incorporated herein by reference, or the fragment of amino acids 13-118 of TACI extracellular domain disclosed in Chinese Patent Publication No. CN101323643A.

The immunoglobulin of the TACI-immunoglobulin fusion protein provided by the present disclosure is preferably IgG1, which may comprise a heavy chain constant region, for example, of human. The preferred IgG1 heavy chain constant region of the present disclosure comprises an IgG1 Fc fragment containing CH2 and CH3 domains, which may be a wild-type IgG1 Fc fragment or a mutated IgG1 Fc fragment.

In the term "fragment of human immunoglobulin constant region", the immunoglobulin is preferably IgG1, which may comprise a heavy chain constant region, for example, of human. The preferred "fragment of human immunoglobulin constant region" of the present disclosure is an amino acid fragment comprising a portion of the hinge region domain, a CH2 domain and a CH3 domain. In some more preferred embodiments, the "fragment of human immunoglobulin constant region" described in the present disclosure has an amino acid sequence set forth in SEQ ID NO: 2, or an amino acid sequence having at least 90%, at least 91%, at least 92%, at least 93%, at least 95%, at least 96%, at least 97%, at least 98%, or at least 99% identity to SEQ ID NO: 2. In some more preferred embodiments, the "fragment of human immunoglobulin constant region" has an amino acid sequence set forth in SEQ ID NO:3.

The term "amino acid" involved in the present disclosure is understood in the broadest sense, and it is a general term for a class of organic compounds containing amino and carboxyl. Preferably, the amino acid involved in the present disclosure is the main unit of proteins constituting living organisms, which includes, but is not limited to: glycine, alanine, valine, leucine, isoleucine, methionine, proline, tryptophan, serine, tyrosine, cysteine, phenylalanine, asparagine, glutamine, threonine, aspartic acid, glutamic acid, lysine, arginine and histidine.

The three-letter codes and one-letter codes of amino acids used in the present disclosure are as described in J.biol.chem, 243, p3558 (1968). There are many ways of numbering amino acid positions, such as Kabat numbering system, EU numbering system, and sequence numbering. In the present disclosure, the amino acid positions are numbered using "sequence numbering", for example, the "position 3, 8, 14, 15, 17, 110, 111 or 173 of SEQ ID NO: 2" in the present disclosure refers to the 3rd amino acid, the 8th amino acid, and so on of SEQ ID NO: 2. For example, the "P3T" in the present disclosure refers to the mutation of the 3rd amino acid of SEQ ID NO:2 from the previous "P" to "T", and "L8P" refers to the mutation of the 8th amino acid of SEQ ID NO:2 from the previous "L" to "P", and so on.

As an alternative embodiment, the immunoglobulin constant region provided by the present disclosure can be introduced with one or more amino acid modifications, such as substitution (i.e., mutation), addition (i.e., insertion) or deletion.

The term "Telitacicept" used in the present disclosure refers to a TACI-Fc fusion protein, having an INN name of Telitacicept and an amino acid sequence set forth in SEQ ID NO: 4 or referring to https://extranet.who.int/soinn/mod/page/view.php?id=137&inn_n=10932.

The TACI-Fc fusion protein of the present disclosure may be administered by various routes, including but not limited to oral administration, intravenous injection, intramuscular injection, intraarterial injection, intramedullary injection, intraperitoneal injection, intrathecal injection, intracardiac administration, transdermal administration, transdermal administration, topical administration, subcutaneous administration, intranasal administration, enteral administration, sublingual administration, vaginal administration, rectal administration, etc.

The term "treatment" involved in the present disclosure is related to a given disease or condition, including but not limited to: inhibiting the disease or condition, such as preventing the development of the disease or condition; alleviating the disease or condition, such as causing the regress of the disease or condition; or ameliorating the symptoms caused by the disease or condition, such as alleviating, preventing or treating the symptoms of the disease or condition.

The embodiments of the present disclosure will be described in detail below in conjunction with examples. However, those skilled in the art will understand that the following examples are only intended to illustrate the present disclosure, and should not be construed as limiting the scope of the present disclosure.

### Example 1 Clinical trial of Telitacicept in the treatment of Sjogren's syndrome

### 1. Research methods

This study was a multi-center, randomized, double-blind, placebo-controlled phase II clinical study. Subjects are confirmed, anti-SSA antibody-positive patients with primary Sjogren's syndrome (pSS), who were required to have ESSDAI≥5 during the screening period. The trial was divided into 2 parts, including a screening period and a double-blind treatment period. The screening period was from Day -28 to Day -1, during which the subjects who met the inclusion criteria but not the exclusion criteria were randomly assigned to the placebo group, the Telitacicept 160 mg group and the Telitacicept 240 mg group according to the ratio of 1:1:1. The double-blind treatment period was from Day 0 to Day 168 (the 24th week), during which the drug was administered once a week for a total of 24 times.

| **Number** | **Name** | **Administration method** |
|---|---|---|
| **1** | Placebo group | Administration method and dosage: A lyophilized preparation for injection with no active ingredients (including histidine hydrochloride, arginine hydrochloride, mannitol and sucrose); subcutaneous injection; once a week, 24-week course of treatment. |
| **2** | Telitacicept 160 mg group | Administration method and dosage: A lyophilized preparation for injection; subcutaneous injection; once a week, 160 mg each time, 24-week course of treatment. |
| **3** | Telitacicept 240 mg group | Administration method and dosage: A lyophilized preparation for injection; subcutaneous injection; once a week, 240 mg each time, 24-week course of treatment. |

### 2. Included subjects

Included were patients who met the 2016 ACR/EULAR classification criteria for primary Sjogren's syndrome and had at least one of the symptoms of dry eyes or dry mouth, i.e., positive for at least one of the following:
- daily unbearable dry eyes for more than 3 months;
- repeated gritty sensation in the eyes;
- requiring use of artificial tears 3 or more times a day;
- daily dry mouth for more than 3 months;
- requiring frequent drinking assistance when swallowing dry food, positive for anti-SSA antibody, and ESSDAI≥5.

### 3. Evaluation criteria

### 3.1 Efficacy indicator

### 3.1.1 Main efficacy indicators

- Changes in ESSDAI score at the 24th week compared with the baseline.

### 3.1.2 Secondary efficacy indicators

- Changes in ESSDAI score at the 12th week compared with the baseline;
- Changes in ESSPRI scores at the 12th and 24th weeks compared with the baseline;
- Changes in disease activity of physicians' overall assessment at the 12th and 24th weeks compared with the baseline;
- Changes in disease activity of patients' overall assessment at the 12th and 24th weeks compared with the baseline;
- Changes in SF-36 at the 12th and 24th weeks compared to the baseline;
- Changes in MFI-20 at the 12th and 24th weeks compared to the baseline;
- Unstimulated whole saliva (UWS) flow rate;
- The scale corresponding to the leading edge of the wetted part of the filter paper strip at 5 min;
- Immunological indicators: IgG, IgA, IgM, complement (C3, C4), the total number of B lymphocytes (CD19+), the number of CD4+ T cells, and the number of CD8+ T cells.

### 3.2 Safety evaluation

- Adverse events;
- Laboratory tests;
- Vital signs;
- Chest x-ray;
- **Electrocardiogram;**
- **Immunogenicity.**

### 4. Statistical methods

This study used SAS 9.4 software for analysis. All statistical tests were two-sided tests, and a P value of≤0.05 was considered statistically significant. Continuous variables were described by means, standard deviations, medians, minimum and maximum values, and count and rank data were described by frequencies and percentages.

There are 3 analysis sets in this study:
- Full analysis set (FAS): A full analysis set refers to the collection of all cases that have been randomized and used the test drug at least once.
- Per-protocol set (PPS): A per-protocol set is a dataset generated by subjects who are in sufficient compliance with the trial protocol, and the compliance includes the treatment received, the accessibility of the primary endpoint indicator measurement, the absence of major violations of the trial protocol, etc.
- Safety set (SS): It is an actual dataset from cases that received at least one treatment and had safety indicators recorded. For the incidence of adverse events, the number of cases in the safety set is used as the denominator.

### 5. Demographics and baseline characteristics

This trial screened 57 patients, and 42 cases were randomly enrolled, of which 30 cases completed the 24-week trial observation and 12 cases withdrew from the trial early. Finally, 42 cases were included in the full analysis set (FAS), 30 cases were included in the per-protocol set (PPS), and 42 cases were included in the safety set (SS). In the placebo group, there were 14 cases of FAS, 10 cases of PPS, and 14 cases of SS. In the 160 mg group, there were 14 cases of FAS, 12 cases of PPS, and 14 cases of SS. In the 240 mg group, there were 14 cases of FAS, 8 cases of PPS, and 14 cases of SS.

At the baseline, the following characteristics of the subjects in the 240 mg group (N=14), 160 mg group (N=14) and placebo group (N=14) were all comparable (P>0.05), including age, height, weight, BMI, gender, ethnicity, occupation, marriage status, reproductive status, smoking history, drug allergy history, primary Sjogren's syndrome course, primary Sjogren's syndrome diagnostic score, primary Sjogren's syndrome manifestations, primary Sjogren's syndrome treatment history, vital signs, virology examination, thyroid function test, immunological indicators, ESSDAI total score, investigator's overall assessment of the disease, ESSPRI total score and scores of each dimension, subject's overall assessment of the disease, SF-36 total score and scores of each dimension, MFI-20 total score and scores of each dimension, autoantibody test, BLyS concentration, and APRIL concentration.

### 6. Efficacy results

### 6.1 Primary efficacy endpoint

The main efficacy endpoint of this study was the change in ESSDAI score compared with the baseline at the 24th week. The differences among groups were compared using mixed-effects model for repeated measures (MMRM) during the analysis, and missing data were not filled. The analysis results of FAS show that the change of the ESSDAI score compared with the baseline at the 24th week after treatment and 95% CI are as follows: in the placebo group (N=14), -0.3 [95% CI: -2.1-1.6]; in the 160 mg group (N=14), -4.0 [95%Cl:-5.7--2.3]; in the 240 mg group (N=14), -3.1 [95%CI:-5.2∼-1.0], According to MMRM comparison, the 160 mg group and the placebo group were statistically significantly different (P=0.004), and the 240 mg group and the placebo group were statistically significantly different (P=0.044). The analysis results of PPS show that the changes of ESSDAI score compared with the baseline at the 24th week after treatment and 95% CI are as follows: in the placebo group (N=10), -0.2 [95% CI: -1.8-1.4]; in the 160 mg group (N=12), -3.9 [95%CI: -5.4∼-2.5]; in the 240 mg group (N=8), -3.1 [95%CI: -4.8∼-1.3]. According to MMRM comparison, the 160 mg group and the placebo group were statistically significantly different (P<0.001), and the 240 mg group and the placebo group were statistically significantly different (P=0.019).

### 6.2 Secondary efficacy endpoints

### 6.2.1. Changes in ESSDAI score compared with the baseline at the 12th week

The change in ESSDAI score compared with the baseline at the 12th week is one of the secondary efficacy indicators in this study. The differences among groups were compared using mixed-effects model for repeated measures (MMRM) during the analysis, and missing data were not filled. The analysis results of FAS show that the change of the ESSDAI score compared with the baseline at the 12th week after treatment and 95% CI are as follows: in the placebo group (N=14), 0.7 [95%CI: -1.0-2.4]; in the 160 mg group (N=14), -3.8 [95%CI: -5.5∼-2.1]; in the 240 mg group (N=14), -2.5 [95%CI: -4.3--0.6]. According to MMRM comparison, the 160 mg group and the placebo group were statistically significantly different (P<0.001), and the 240 mg group and the placebo group were statistically significantly different (P=0.013). The analysis results of PPS show that the changes of ESSDAI score compared with the baseline at the 12th week after treatment and 95% CI are as follows: in the placebo group (N=10), -0.5 [95%CI: -2.1-1.1]; in the 160 mg group (N=12), -3.8 [95%CI: -5.2∼-2.3]; in the 240 mg group (N=8), -2.9 [95%CI: -4.7∼-1.2]. According to MMRM comparison, the 160 mg group and the placebo group were statistically significantly different (P=0.003), and the 240 mg group and the placebo group were statistically significantly different (P=0.045).

### 6.2.9. Immunological indicators

Immunological indicators include: IgG, IgA, IgM, complement (C3, C4), the total number of B lymphocytes (CD19+), the number of CD4+ T cells, and the number of CD8+ T cells. The changes in immunological indicators after treatment compared with the baseline were compared among groups using analysis of variance. When P was ≤0.05 in analysis of variance, pairwise comparison among groups was conducted using LSD-t test. The actual data in FAS was used for analysis.

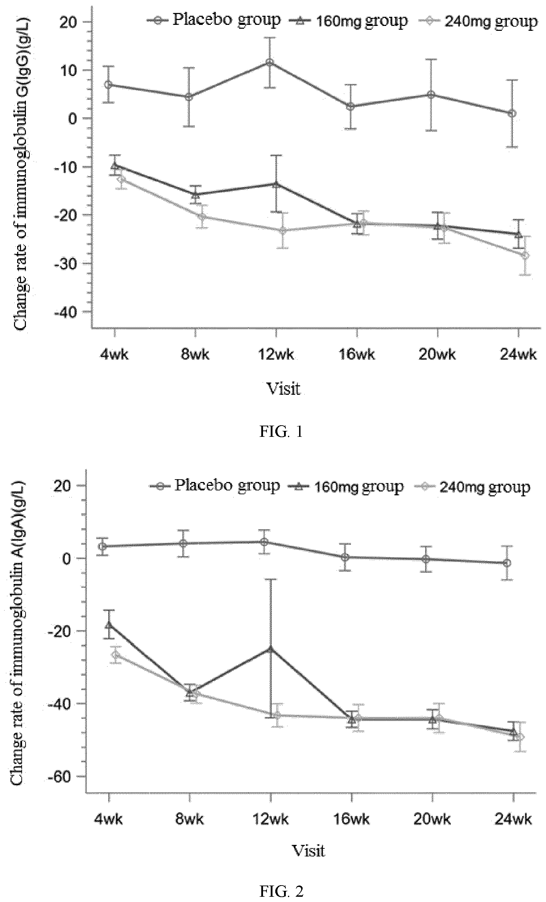

Compared with the placebo group, the levels of IgG, IgA, and IgM in the Telitacicept treatment group decreased significantly since the 4th week, and the decrease lasted until the 24th week. The change rates of IgG levels in the placebo group, 160 mg group, and 240 mg group compared with the baseline at the 24th week are as follows: 1.02±21.855, -23.91±10.199 and -28.40±11.219, where the 160 mg group and the placebo group were statistically significantly different (P<0.001), and the 240 mg group and the placebo group were statistically significantly different (P<0.001). The change rates of IgA levels in the placebo group, 160 mg group, and 240 mg group compared with the baseline at the 24th week are as follows: -1.29±14.749, -47.55±8.790 and -49.18±11.294, where the 160 mg group and the placebo group were statistically significantly different (P<0.001), and the 240 mg group and the placebo group were statistically significantly different (P<0.001). The change rates of IgM levels in the placebo group, 160 mg group, and 240 mg group compared with the baseline at the 24th week are as follows: -7.87±19.062, -55.74±11.010 and -61.61±8.692, where the 160 mg group and the placebo group were statistically significantly different (P<0.001), and the 240 mg group and the placebo group were statistically significantly different (P<0.001). The diachronic analysis of the change rate of IgG, IgA, and IgM levels in each group compared with the baseline is shown in FIGs. 1-3.

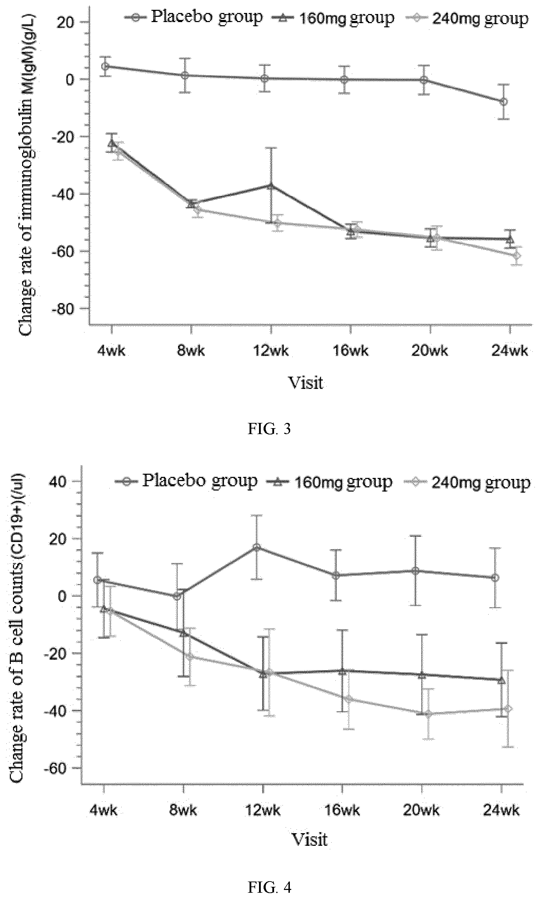

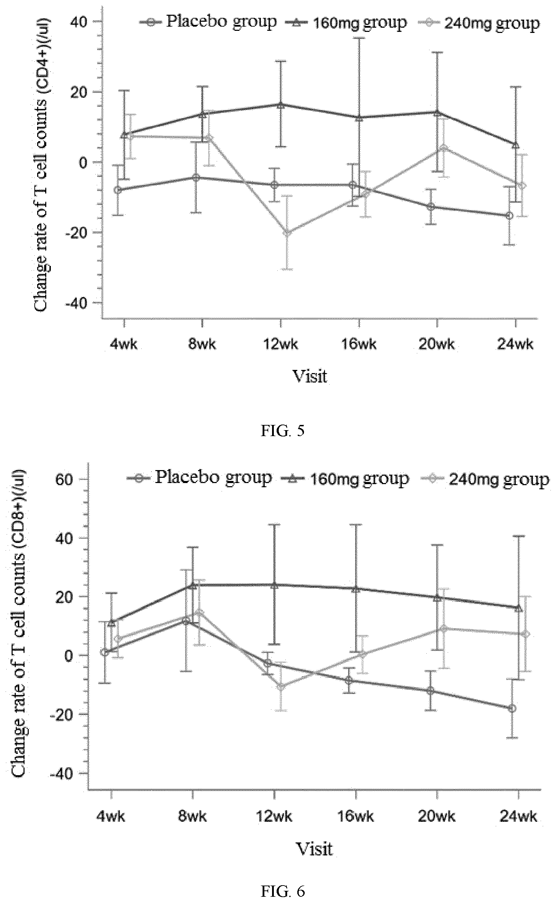

Compared with the placebo group, the number of CD19+ B cells in the Telitacicept treatment group showed a significant downward trend. The change rates of CD19+ B cell counts in the placebo group, 160 mg group, and 240 mg group compared with the baseline at the 24th week are as follows: 6.31±32.826, -29.26±44.698 and -39.36±37.836, where the 160 mg group and the placebo group were statistically significantly different (P=0.044), and the 240 mg group and the placebo group were statistically significantly different (P=0.021). The diachronic analysis of the change rate of CD19+ B cell counts in each group compared with the baseline is shown in FIGs. 4-6.

### 6.3 Safety Results

Only one subject in the placebo group had serious adverse events, manifested as hypoalbuminemia, herpes zoster, pulmonary tuberculosis, infectious pneumonia, and aggravated Sjogren's syndrome, which were relieved after treatment.

Only 2 subjects in the 240 mg group experienced severe and more severe (CTCAE grade 3-5) adverse events, which were acute pyelonephritis and leukopenia, respectively.

### 6.4 Conclusion

Compared with the placebo group, the Telitacicept treatment significantly improved the ESSDAI score and MF-20 score of the patients with pSS at the 24th week; and Telitacicept showed good safety in the patients with pSS.

The above description is only for preferred embodiments by way of example only and without limitation to the combination of features necessary for carrying the present disclosure into effect. The headings provided herein are not intended to limit the various embodiments of the present disclosure. **Terms such as "including", "comprising" and "containing" are not intended to be limiting. In addition, unless otherwise indicated, the singular form "a", "an", or "the" includes plural references, as well as "or" means "and/or".** Unless otherwise defined herein, all technical and scientific terms used herein have the same meaning as commonly understood by those skilled in the art.

All publications and patents mentioned in the present application are incorporated herein by reference. Various modifications and variations of the methods and compositions described in the present disclosure without departing from the scope and spirit of the present disclosure will be apparent to those skilled in the art. Although the present disclosure has been described by using specific preferred embodiments, it should be understood that the claimed invention should not be unduly limited to these specific embodiments. In fact, many variations of the described modes for carrying out the invention that are obvious to those skilled in the art are intended to be included within the scope of the appended claims.

## Claims

1. A method for treating Sjogren's syndrome (SS), comprising administering a therapeutically effective amount of a TACI-Fc fusion protein to a patient with Sjogren's syndrome, wherein the TACI-Fc fusion protein comprises:
(i) a TACI extracellular region or a fragment thereof binding to Blys and/or APRIL; and
(ii) a fragment of human immunoglobulin constant region.

2. The method according to claim 1, wherein the TACI extracellular region or the fragment thereof binding to Blys and/or APRIL comprises an amino acid sequence set forth in SEQ ID NO: 1.

3. The method according to claim 2, wherein the human immunoglobulin is IgG1, or the fragment of human immunoglobulin constant region comprises an amino acid sequence set forth in SEQ ID NO: 2 or an amino acid sequence having at least 90%, at least 91%, at least 92%, at least 93%, at least 95%, or at least 96% identity to SEQ ID NO: 2.

4. The method according to claim 3, wherein the fragment of human immunoglobulin constant region comprises one or more modifications of amino acid at positions 3, 8, 14, 15, 17, 110, 111 or 173 of SEQ ID NO: 2.

5. The method according to claim 4, wherein the modification is substitution, deletion or insertion of amino acid.

6. The method according to claim 5, wherein the substitution is selected from the group consisting of P3T, L8P, L14A, L15E, G17A, A110S, P111S and A173T.

7. The method according to claim 6, wherein the fragment of human immunoglobulin constant region comprises an amino acid sequence set forth in SEQ ID NO: 3.

8. The method according to claim 1, wherein the TACI-Fc fusion protein has an amino acid sequence set forth in SEQ ID NO: 4.

9. The method according to claim 8, wherein the TACI-Fc fusion protein is Telitacicept.

10. The method according to claim 8 or 9, wherein the Sjogren's syndrome comprises primary Sjogren's syndrome or secondary Sjogren's syndrome.

11. The method according to claim 8 or 9, wherein the TACI-Fc fusion protein is administered at a single dose of about 0.1 to 10 mg/kg.

12. The method according to claim 8 or 9, wherein the TACI-Fc fusion protein is administered at a single dose of 160 to 240 mg, preferably 160 mg or 240 mg.

13. The method according to claim 12, wherein the TACI-Fc fusion protein is administered subcutaneously, intramuscularly or intravenously, or at the thigh, abdomen or upper arm.

14. The method according to claim 12, wherein the TACI-Fc fusion protein is administered 2-4 times at intervals of one month.

15. The method according to claim 14, wherein the TACI-Fc fusion protein is administered at a frequency of once a week.

16. The method according to claim 14, wherein the administration lasts for about 2-50 weeks.
